**Europäisches Patentamt**

**European Patent Office**

**Office européen des brevets**

(19)

(11) Veröffentlichungsnummer: **0 239 872**
**B1**

(12) **EUROPÄISCHE PATENTSCHRIFT**

(45) Veröffentlichungstag der Patentschrift:
**31.10.90**

(51) Int. Cl.⁵: **C07C 205/37**, C07C 201/12

(21) Anmeldenummer: **87103917.8**

(22) Anmeldetag: **17.03.87**

(54) Verfahren zur Herstellung von 4-Nitrophenetol.

(30) Priorität: **29.03.86 DE 3610707**

(43) Veröffentlichungstag der Anmeldung:
**07.10.87 Patentblatt 87/41**

(45) Bekanntmachung des Hinweises auf die Patenterteilung:
**31.10.90 Patentblatt 90/44**

(84) Benannte Vertragsstaaten:
**DE FR GB IT SE**

(56) Entgegenhaltungen:
EP-A- 0 065 770
CH-A- 626 866
DE-A- 3 307 164
US-A- 1 619 368
US-A- 2 166 917

(73) Patentinhaber: **BAYER AG,**
**D-5090 Leverkusen 1 Bayerwerk(DE)**

(72) Erfinder: **Käsbauer, Josef, Dr., Gartenfeld 37,**
**D-5632 Wermelskirchen 2(DE)**
Erfinder: **Wedemeyer, Karlfried, Dr., Bilharzstrasse 7,**
**D-5000 Köln 80(DE)**

## Beschreibung

Die Erfindung betrifft ein Verfahren zur Herstellung von 4-Nitrophenetol, das praktisch frei ist von 4-Nitrophenol und 4,4'-Dichlorazoxybenzol.

Es ist bekannt, 4-Nitrophenetol herzustellen durch Umsetzung von 4-Chlornitrobenzol mit Alkalimetallhydroxiden und Ethanol (C. Willgerodt, Chem. Ber. 15 (1882), 1002). Nachteilig bei diesem Herstellungsverfahren ist die geringe Reaktionsgeschwindigkeit sowie die Bildung von Azoxyverbindungen durch reduktive Dimerisierung des 4-Nitrophenetols, vor allem des 4-Chlornitrobenzols.

Um die Bildung von Azoxyverbindungen zu unterdrücken, wurde versucht die Umsetzung in Gegenwart von Luft oder anderen Oxidationsmitteln durchzuführen (siehe z.B. die US-PS 2 545 597; die DDR-Patentschrift 8 1113, Indian Chem. Mfr. 9(3), 21 bis 22 (1971), Khim.-Farm. Zh. 8(6), 29 bis 30 (1974), Yakhak Hoe Chi, 19(2), 101 bis 110 (1975), Khim. Ind. (Sofia), 10, 451 bis 453 (1981), Japan Kokai JP 49/126634 (1974) sowie J. Org. Chem. 45 (11), 2263 bis 2264 (1980)).

Die Verfahren in den genannten Veröffentlichungen haben den Nachteil, daß sie entweder bei sehr langer Reaktionszeit eine ungenügende Ausbeute ergeben (JP 49/126634: 33 Stunden Reaktionszeit, 88,2 % Ausbeute) oder bei kurzer Reaktionszeit dennoch Azoxyprodukte bilden (z.B. Khim. Ind. (Sofia) 10, 451 bis 453 (1981): 7 Stunden, 0,6 % 4,4'-Dichlorazoxybenzol, bis 90 % Ausbeute) oder daß bei langer Reaktionszeit noch 4-Nitrophenol entsteht (z.B. US-PS 2 545 597: 24 Stunden, 5 % Nitrophenol). Das gebildete Nitrophenol stört sowohl bei der Weiterverarbeitung zum 4-Nitrophenetol als auch durch seine Wasserlöslichkeit.

Weiterhin ist bekannt, die Umsetzung von 4-Chlornitrobenzol mit Alkalimetallhydroxiden und Ethanol zu 4-Nitro-phenetol auch ohne den Zusatz von Oxidationsmittel durchzuführen. So wird in der DE-OS 26 34 419 ein Verfahren zur Herstellung von aromatischen Ethern beschrieben, das dadurch gekennzeichnet ist, daß man ein mit Wasser unmischbares acetyliertes aromatisches Halogenid, worin das Halogenatom aus einem Kernsubstituenten besteht, mit einer organischen Hydroxyverbindung in Gegenwart eines wäßrigen Alkalis und eines Phasentransferkatalysators umsetzt. Gemäß Beispiel 6 der DE-OS werden unter Rückflußbedingungen bei 90 %iger Ausbeute an 4-Nitrophenetol noch 10 % 4,4'-Dichlorazoxybenzol erhalten. Das Auftreten diese Nebenprodukts erschwert die Aufarbeitung des 4-Nitrophenetols und stört die weitere Umsetzung des 4-Nitrophenetols.

In der europäischen Patentanmeldung 65 770 wird die Herstellung von p-Nitrophenetol aus p-Chlornitrobenzol und Ethanol durch Erwärmen unter Zugabe von Alkalimetal hydroxiden und Phasentransferkatalysatoren beschrieben, wobei die Umsetzung bei Temperaturen von 60 bis 80°C durchgeführt wird. Gemäß Beispiel 1 der europäischen Patentanmeldung wird bei einer Temperatur von 70°C das 4-Nitrophenetol in 94,8 %iger Ausbeute erhalten. Nachteilig bei diesem Verfahren sind die großen Mengen an Phasentransferkatalysator mit 8,5 Gew.-% und die eng begrenzte Temperaturführung. So wird im Vergleichsbeispiel gezeigt, daß sich unter Rückflußbedingungen 13 % 4,4'-Dichlorazoxybenzol und 10 % 4,4'-Diethoxyazoxybenzol neben 2 % 4-Nitrophenol bilden. In dem Beispiel 1 der europäischen Patentanmeldung wird die Bedeutung einer strengen Temperaturkontrolle nochmals betont, da ausdrücklich darauf hingewiesen wird, daß die Reaktionstemperatur während der gesamten Reaktionsdauer nicht über 70°C ansteigen darf.

Auf eine strenge Temperaturkontrolle wird auch bei dem Verfahren gemäß der DE-OS 33 07 164 geachtet. In der genannten DE-OS wird nämlich auf Seite 9 im letzten Absatz bei dem Herstellungsverfahren von o- oder p-Nitrophenetol hervorgehoben, daß bei Temperaturen über 80°C sich einige Azoxyverbindungen zu bilden beginnen, weshalb es äußerst zweckmäßig sei, diese obere Temperaturgrenze nicht zu übersohreiten.

Es wurde nun ein Verfahren zur Herstellung von 4-Nitrophenetol durch Umsetzung von 4-Chlornitrobenzol mit Ethanol und Alkalimetallhydroxiden in Gegenwart von Phasentransferkatalysatoren gefunden, das dadurch gekennzeichnet ist, daß man die Umsetzung bei Temperaturen von 60 bis 95°C unter Druck in Gegenwart von mit Inertgasen verdünnten sauerstoffhaltigen Gasen durchführt, wobei zu Anfang der Umsetzung 0,2 bis 1,0 Mol Alkalimetallhydroxid, bezogen auf 1 Mol 4-Chlornitrobenzol, pro Stunde dem Reaktionsgemisch zudosiert werden.

Als Phasentransferkatalysatoren seien die üblichen quaternären organischen Ammoniumsalze, wie Tetrabutylammoniumbromid, Benzyltripropylammoniumchlorid, Benzyldodecyldimethylammoniumchlorid, Benzyltriethylammoniumchlorid und/oder Benzyltrimethylammoniumchlorid genannt. Bevorzugt sei das Benzyltrimethylammoniumchlorid genannt.

Die Phasentransferkatalysatoren werden im allgemeinen in einer Menge von etwa 1 bis 5 Gew.-%, bevorzugt 1,5 bis 3,5 Gew.-%, bezogen auf das Einsatzprodukt, eingesetzt. In vorteilhafter Weise können die Phasentransferkatalysatoren vor Beginn der eigentlichen Reaktion im Reaktionsgefäß hergestellt werden. So ist es beispielsweise möglich das Benzyltrimethylammoniumchlorid durch Umsetzung von Trimethylamin und Benzylchlorid in Ethanol herzustellen.

Als Alkalimetallhydroxide kommen beispielsweise für das erfindungsgemäße Verfahren in Frage das Natrium- und/oder Kaliumhydroxid. Die Alkalimetallhydroxide können sowohl in fester Form als auch in wäßriger oder alkoholischer Lösung dem Reaktionsgemisch zudosiert werden. So ist beispielsweise eine 50 %ige wäßrige Lösung an Natriumhydroxid bevorzugt. Die Menge an Alkalimetallhydroxiden beträgt im allgemeinen etwa 2 bis 5 Mol, besonders bevorzugt 2,5 bis 4 Mol Alkalimetallhydroxid pro Mol 4-Chlornitrobenzol.

Um die Bildung vor allem von 4,4'-Dichlorazoxybenzol durch reduktive Dimerisierung des 4-Chlornitrobenzols zu vermeiden, ist darauf zu achten, daß das Alkalimetallhydroxid, beispielsweise die Natronlauge, nicht beliebig schnell dem Reaktionsgemisch zugegeben wird. Besonders der Anfangsbereich der Umsetzung ist bei kleinen Phasentransferkonzentrationen kritisch. So sollten in den ersten 1 1/2 bis 2 1/2 Stunden, bevorzugt in den ersten 2 Stunden, nur etwa 0,2 bis 1,0 Mol Alkalimetallhydroxid pro Mol Einsatzprodukt und pro Stunde zugegeben werden. Bevorzugt ist eine Menge von 0,3 bis 0,6 Mol Alkalimetallhydroxid pro Mol Einsatzprodukt und pro Stunde. Im weiteren Verlauf der Reaktion ist die zugegebene Menge an Alkalimetallhydroxid nicht mehr kritisch und kann über 1 Mol pro Stunde gesteigert werden, so daß die gesamte Menge an Alkalimetallhydroxid in etwa 4 bis 6 Stunden zudosiert wird.

Unterstützt wird das Aufrechterhalten einer niedrigen Basenkonzentration in dem Reaktionsgemisch durch ein, auch bei technischen Rührgeschwindigkeiten, effektives Rühren des Reaktionsgemisches, vor allem der Phasengrenze.

Als sauerstoffhaltige Gase eignen sich sowohl Luft als auch Sauerstoff. Diese werden bei dem erfindungsgemäßen Verfahren im Gemisch mit Inertgasen, wie Stickstoff, dem Reaktionsgemisch aufgepreßt. Nach dem erfindungsgemäßen Verfahren arbeitet man bei einem Druck des sauerstoffhaltigen Gases von etwa 1 bis 10 bar, bevorzugt 2 bis 8 bar. Dabei richtet sich der Druck des aufgepreßten sauerstoffhaltigen Gases nach dem Verhältnis von Ansatzgröße zu Autoklaven-Volumen und kann leicht durch Vorversuche ermittelt werden. So wird beispielsweise bei einem 1 Mol Ansatz in einem 0,7 l Autoklaven ein Anfangsdruck von 1 bis 4 bar, bevorzugt von 1,5 bis 3,5 bar Luft bei 83°C benötigt.

Die Menge an Inertgas, die dem sauerstoffhaltigen Gas zugemischt wird, kann in weiten Grenzen variiert werden. Es wird mindestens soviel Inertgas zugegeben, daß keine explosiven Gemische entstehen. Dies kann ebenfalls leicht durch entsprechende Versuche ermittelt werden.

Das Ethanol kann nach dem erfindungsgemäßen Verfahren sowohl in reiner Form als auch in bevorzugter Weise als technische Ware mit einem Gehalt von etwa 95 Gew.-% eingesetzt werden. Man benötigt etwa 1,0 bis 1,6 Mol Ethanol, bevorzugt 1,1 bis 1,4 Mol Ethanol, pro Mol Einsatzprodukt.

Die Reaktionstemperatur beträgt etwa 60 bis 95°C, bevorzugt 75 bis 90°C, besonders bevorzugt 80 bis 85°C.

Bei dem erfindungsgemäßen Verfahren werden im Vergleich zu den bekannten Verfahren wesentlich geringere Mengen an Phasentransferkatalysatoren benötigt, wodurch sich das erfindungsgemäße Verfahren besonders wirtschaftlich gestaltet. Der Phasentransferkatalysator kann dann in der verbleibenden geringen Konzentration elegant ohne technische Probleme aus dem Abwasser entfernt werden.

Das erfindungsgemäße Verfahren ist darüber hinaus nicht empfindlich gegen höhere Reaktionstemperaturen. Auch bei Temperaturen über 80°C bilden sich überraschenderweise praktisch keine Azoxynebenprodukte. Dadurch wird die Aufarbeitung, Reinigung und Weiterverarbeitung des 4-Nitro-phenetols erheblich erleichtert. Das 4-Nitrophenetol läßt sich ohne Emulsionsbildung in der Schmelze abtrennen. Außerdem bilden sich trotz höherer Reaktionstemperatur keine nennenswerte Mengen an 4-Nitrophenol, was ebenfalls die Aufarbeitung des Reaktionsproduktes erleichtert.

Im Gegensatz zu den bekannten Verfahren wird beim erfindungsgemäßen Verfahren nicht in einem Luftstrom gearbeitet, sondern in einem geschlossenen System. Dadurch wird die Gefahr einer explosiven Zone aus einem Ethanol-Luftgemisch im Abgasstrom vermieden. Das erfindungsgemäße Verfahren trägt also zu einer höheren Verfahrenssicherheit bei.

Beispiel 1

In einem 1 l-Autoklaven wurden 157,6 g, 1 Mol 4-Chlornitrobenzol, 67,8 g, 1,4 Mol Ethanol (95 %ig) und 5,6 g Benzyltrimethylammoniumchlorid (3 Mol-%) bei 83°C vorgelegt. Man drückte mit Preßluft auf 2 bar Innendruck auf und pumpte 210 ml 50 %ige wäßrige Natronlauge mit folgender Geschwindigkeit zu: 1. Stunde: 20 ml, 2 Stunde: 30 ml, dann 50 ml/h. Der Innendruck stieg auf 2,8 bar. Es wurde 6 Stunden bei 83°C nachgerührt. Nach dem Entspannen gab man 220 ml heißes Wasser zu, trennte die organische Phase ab und wusch sie zweimal mit 70 ml heißem Wasser. Beim Abkühlen kristallisierte das Produkt in hellgelber Farbe aus. Die vereinigten Wasserphasen wurden andestilliert und die Raumtemperatur von etwas ausgefallenem Produkt abgesaugt. Ausbeute: 165 g, 98,7 % d.Th., Schmp. 57-58°C, Reinheit 99,25%, 0,75 % Ausgangsprodukt. Das erhaltene Produkt ist frei von 4,4'-Dichlorazoxybenzol und 4-Nitrophenol.

Das Abwasser enthielt 0,1 bis 0,2 g 40-Nitrophenol.

Beispiel 2

In einem 0,7 l-Autoklaven wurden 157,6 g 4-Chlornitrobenzol, 67,8 g Ethanol (95 %ig) und 4,6 g Benzyltrimethylammoniumchlorid (2,5 Mol-%) bei 83°C vorgelegt. Man drückte mit Preßluft auf 3,3 bar und mit $N_2$ auf 6,5 bar Innendruck auf. Dann wurden 160 ml 50 %ige wäßrige Natronlauge wie folgt zugepumpt: 1. Stunde: 20 ml, 2. Stunde: 30 ml, 3. Stunde: 40 ml, dann 50 ml/h. Der Innendruck stieg auf 10 bar. Man rührte 5-6 Stunden bei 83°C weiter, entspannte und gab 170 ml heißes Wasser zu. Die organische Phase wurde abgetrennt und zweimal mit 70 ml Wasser bei 70°C gewaschen. Die vereinigten Wasserphasen wurden andestilliert und bei Raumtemperatur abgesaugt. Ausbeute: 165,5 g, 99 % d. Th., Reinheit 97 %. Das Produkt ist frei von Azoxynebenprodukten und von 4-Nitrophenol.

Beispiel 3

In einem 0,7 l Autoklaven wurden 1 Mol 4-Chlornitrobenzol, 1,2 Mol Ethanol (95 %ig) und 10 g Benzyltriethylammoniumchlorid bei 83°C und 7 bar Luftdruck vorgelegt. Man pumpte 210 ml 50 %ige wäßrige Natronlauge in 3 Stunden zu und rührte 8 Stunden weiter. Der Druck stieg auf 10 bar an. Aufarbeitung wie in Beispiel 1. Das Produkt enthielt 1,2 % Ausgangsprodukt, kein Azoxynebenprodukt und kein 4-Nitrophenol.

Beispiel 4 (Vergleichsbeispiel)

Beispiel 1 wurde wiederholt mit der Ausnahme, daß kein sauerstoffhaltiges Gas (Luft) aufgedrückt wurde. Nachdem der Autoklav geschlossen war, wurde auf 83°C aufgeheizt. Dabei stellte sich ein Eigendruck von 1 bar ein. Nach Aufarbeitung erhielt man ein Produkt, das noch 1,2 % an Ausgangsprodukt enthielt und 5,6 % an 4,4'-Dichlorazoxybenzol.

Beispiel 5 (Vergleichsbeispiel)

Beispiel 2 wurde wiederholt mit der Ausnahme, daß bei 3 Mol-% Benzyltrimethylammoniumchlorid 210 ml 50 %ige Natronlauge in 3 Stunden zugepumpt wurden. Das Produkt enthielt 0,3 % Ausgangsprodukt und 0,2 % 4,4'-Dichlorazoxybenzol.

**Patentansprüche**

1. Verfahren zur Herstellung von 4-Nitrophenetol durch Umsetzung von 4-Chlornitrobenzol mit Ethanol und Alkalimetallhydroxiden in Gegenwart von Phasentransferkatalysatoren, dadurch gekennzeichnet, daß man die Umsetzung bei Temperaturen von 60 bis 95°C unter Druck in Gegenwart von mit Inertgasen verdünnten sauerstoffhaltigen Gasen durchführt, wobei zu Anfang der Umsetzung 0,2 bis 1,0 Mol Alkalimetallhydroxid, bezogen auf 1 Mol 4-Chlornitrobenzol, pro Stunde dem Reaktionsgemisch zudosiert werden.

2. Verfahren nach Anspruch 1, dadurch gekennzeichnet, daß man bei einem Druck des sauerstoffhaltigen Gases von 1 bis 10 bar arbeitet.

3. Verfahren nach Ansprüchen 1 und 2, dadurch gekennzeichnet, daß man als sauerstoffhaltiges Gas Luft einsetzt.

4. Verfahren nach Ansprüchen 1 bis 3, dadurch gekennzeichnet, daß man bei Temperaturen von 75 bis 90°C arbeitet.

5. Verfahren nach Ansprüchen 1 bis 4, dadurch gekennzeichnet, daß man bei Temperaturen von 80 bis 85°C arbeitet.

**Claims**

1. Process for the preparation of 4-nitrophenetol by reaction of 4-chloronitrobenzene with ethanol and alkali metal hydroxides in the presence of phase-transfer catalysts, characterized in that the reaction is carried out at temperatures from 60 to 95°C and under pressure in the presence of oxygen-containing gases diluted with inert gases, there being at the start of the reaction 0.2 to 1.0 mol of alkali metal hydroxide metered each hour into the reaction mixture for each 1 mol of 4-chloronitrobenzene.

2. Process according to Claim 1, characterized in that it is carried out under a pressure of the oxygen-containing gas of 1 to 10 bar.

3. Process according to Claims 1 and 2, characterized in that air is used as oxygen-containing gas.

4. Process according to Claims 1 to 3, characterized in that it is carried out at temperatures from 75 to 90°C.

5. Process according to Claims 1 to 4, characterized in that it is carried out at temperatures from 80 to 85°C.

**Revendications**

1. Procédé de production de 4-nitrophénétol par réaction du 4-chloronitrobenzène avec l'éthanol et des hydroxydes de métaux alcalins en présence de catalyseurs de transfert de phases, caractérisé en ce qu'on conduit la réaction à des températures de 60 à 95°C sous pression en présence de gaz contenant de l'oxygène dilués avec des gaz inertes, en ajoutant au mélange réactionnel, par heure, au début de la réaction, 0,2 à 1,0 mole d'hydroxyde de métal alcalin pour 1 mole de 4-chloronitrobenzène.

2. Procédé suivant la revendication 1, caractérisé en ce qu'on opère à une pression du gaz contenant de l'oxygène de 1 à 10 bars.

3. Procédé suivant les revendications 1 et 2, caractérisé en ce qu'on utilise l'air comme gaz contenant de l'oxygène.

4. Procédé suivant les revendications 1 à 3, caractérisé en ce qu'on opère à des températures de 75 à 90°C.

5. Procédé suivant les revendications 1 à 4, caractérisé en ce qu'on opère à des températures de 80 à 85°C.